# EUROPEAN PATENT APPLICATION

(11) **EP 0 931 510 A1**
(43) Date of publication of application: **28.07.1999**
(21) Application number: 99300488.6
(22) Date of filing: 22.01.1999
(51) Int. Cl.: A61B 17/04

(54) **A needle holder to assist in suturing**

(30) Priority: 23.01.1998 US 12783
(71) Applicant: ETHICON ENDO-SURGERY, INC., Cincinnati, OH 45242-2839 (US)
(72) Inventor: Papa, Christopher A., Loveland, Ohio 45140 (US); Swayze, Jeffrey S., Cincinnati, Ohio 45249 (US)
(74) Representative: Fisher, Adrian John

(57) **Abstract**

In accordance with the present invention there is provided a device for assisting a physician in suture procedures using a needle having a suture attached thereto. The device has a handle with distal and proximal ends. Right and left arms extending distally from the handle. The arms have proximal ends attached to the handle and distal ends having grippers attached thereto for gripping and releasing a needle. Each of the grippers has first and second opposing members which move closely adjacent to one another to grip the needle. The first portion is an aperture surrounded by a perimeter wall. The second portion is a clamping member, opposing the aperture. The clamping member is sized such that it can be at least partially inserted within the aperture so that when the gripper is activated, the perimeter wall presses against a first side of the needle at two spaced apart locations, and the clamping member presses against a second opposing side of the needle, between the two spaced apart locations. The device further includes at least one means for moving the distal ends of the arms closely adjacent to one another and for passing the needle from one gripper to the other and thereafter for moving the distal ends of the arms further apart from one another.

## Description

### FIELD OF THE INVENTION

The present invention relates to the field of medicine and more particularly to surgery. More specifically, the present invention relates to surgical devices and methods for suturing bodily tissues both for open surgical procedures and for endoscopic surgical procedures. The present invention especially relates to surgical devices and methods for joining hollow organs, e.g. the anastomosis of the small or large intestines, and blood vessels, for joining them together in end-to-end, end-to-side, or side-to-side fashion.

### BACKGROUND OF THE INVENTION

It is common surgical practice to use bypass grafts to help reestablish coronary artery circulation when a portion of the coronary artery is stenosed. Such a procedure is typically referred to as a Coronary Artery Bypass Graft (CABG) procedure. Typically the graft vessel used in bypassing the stenosed portion of the coronary artery comprises one or more segments of the patient's saphenous vein which is taken from his leg. The saphenous vein is dissected free from the leg, its side branches tied off or ligated, and the vein removed. The vein graft is then washed free of blood, and cut into portions of suitable length. Each portion is then passed to the surgeon who trims the ends of the graft before anastomosing the graft to the aorta and the coronary artery. Other graft vessels such as the radial artery in the arm can also be used. In addition, it is common practice today for the surgeon to redirect one of the internal mammary arteries (IMA) in the chest to the stenosed portion of the left anterior descending (LAD) artery on the heart. The end of the IMA near the patient's diaphragm is transected, the artery is mobilized by dissection and ligation of side branches, and then the end is joined to the LAD, just distal to the blockage. For multiple bypass surgery, a combination of the redirection of the IMA and the grafting of vessels to the diseased coronary arteries is often used.

Some surgeons choose to complete all the proximal anastomoses to the aorta before commencing the distal anastomoses to the coronary arteries. In contrast, others choose to complete the distal anastomoses first. Regardless of the order, when undertaking the distal anastomoses to the coronary artery, it is important that the vessel graft be held steady and adjacent the coronary artery, with a minimum of vascular trauma and a minimum of visual and surgical obstruction by instruments in the narrow operative field.

The speed of performing such anastomoses can become extremely critical as well. Often the coronary artery is occluded during the procedure so that the anastomoses can be performed more easily. It is very important to reconnect the supply of blood to artery as soon as possible in order to minimize or prevent damage to the patient. Blood vessels are now normally anastomosed end-to-end or end-to-side by suturing techniques. Conventionally, to suture two vessels together, a surgeon passes the pointed tip of a curved suturing needle, having a suture attached to the blunt end, through the coronary artery wall from inside the lumen. The needle is then passed through the graft vessel wall from the outside. Then, the surgeon grasps the tip of the needle which has been forced through the tissues with fingers or a needle holder and pulls the needle through the tissues, the suture following the curved path of the needle. Usually a knot or button is present at the trailing end of the suture to anchor the first stitch. After the surgeon has pulled the suture entirely through the tissues to tension the first stitch, he or she then forces the tip of the needle through the coronary artery again, at a location spaced from the first stitch, until the needle again goes through the coronary artery and back out through the graft vessel. Again, he grasps the tip of the needle which has been forced through the tissues, applies tension to the needle pulls the entire suture through the tissues to complete the second stitch. This process is repeated again and again, with the surgeon tensioning the suture after each stitch to draw the tissues together thereby creating a running or continuous stitch, composed of individual thread loops, which extends around the graft vessel.

Needless to say, such suturing techniques are a tedious and time consuming task. Suture anastomoses procedures generally take the skilled surgeon several minutes to complete for each anastomoses. An example of a device which was designed to help a physician in performing suturing can be found in U.S. Patents 5,437,681 issued to Meade et al. on August 1, 1995 and 5,540,705 issued to Meade et al. on July 30, 1996, both of which are hereby incorporated herein by reference. However, there are a number of disadvantages to the device disclosed in those references. In those devices it is the device itself which drives the needle through the tissue. Many physicians do not like this design because they like to have more control of needle placement and feel the resistance of the needle passing through the tissue when doing the procedure. Surgeons want the speed and efficacy offered by the new devices, but also want to maintain the benefits of the traditional suturing techniques.

Presently, there are no known simple, yet foolproof, devices for helping the physician perform the anastomoses more quickly and with greater precision than when using conventional instruments. The present invention provides a device and method which overcomes the shortcomings of the prior art and helps the physician to suture bodily tissues easily and quickly with a single instrument. It is especially useful for performing an easy and quick vascular anastomoses such as for a CABG procedure.

### SUMMARY OF THE INVENTION

In accordance with the present invention there is provided a device for assisting a physician in suture procedures using a needle having a suture attached thereto. The device has a handle with distal and proximal ends. Right and left arms extending distally from the handle. The arms have proximal ends attached to the handle and distal ends having grippers attached thereto for gripping and releasing a needle. Each of the grippers has first and second opposing members which move closely adjacent to one another to grip the needle. The first portion is an aperture surrounded by a perimeter wall. The second portion is a plunger, opposing the aperture. The plunger is sized such that it can be at least partially inserted within the aperture so that when the gripper is activated, the perimeter wall presses against a first side of the needle at two spaced apart locations, and the plunger presses against a second opposing side of the needle, between the two spaced apart locations. The device further includes at least one means for moving the distal ends of the arms closely adjacent to one another and for passing the needle from one gripper to the other and thereafter for moving the distal ends of the arms further apart from one another.

### BRIEF DESCRIPTION OF DRAWINGS

The foregoing and other aspects of the present invention will best be appreciated with reference to the detailed description of the invention in conjunction with the accompanying drawings, wherein:
Figures 1 - 10 depict the sequence of operation of the present invention;
Figure 1 is an isometric view of the present invention showing a curved, surgical needle being placed into the distal end of the device while the loading button is being depressed;
Figure 2 is an enlarged, partial view of the device depicted in Figure 1, with the trigger and handle top removed for clarity;
Figure 3 is an isometric view of the present invention and shows the curved, surgical needle held in the distal end of the device after the loading button has been released, and a portion of the handle top and bottom has been cut away to view the barrel;
Figure 4 is an enlarged, partial view of the device depicted in Figure 3, but with the trigger and handle top removed for clarity;
Figure 5 is an isometric view of the present invention, showing the curved, surgical needle held in the distal end of the right arm and the distal end of the left arm swung laterally apart from the right arm after the trigger has been actuated to the down position;
Figure 6 is an enlarged, partial view of the device depicted in Figure 5, but with the trigger and handle top removed for clarity;
Figure 7 is an isometric view of the present invention showing the curved, surgical needle held in the distal end of both the right and left arms after the trigger has been released to the up position;
Figure 8 is an enlarged, partial view of the device depicted in Figure 7, but with the trigger and handle top removed for clarity;
Figure 9 is an isometric view of the present invention showing the curved, surgical needle held in the distal end of the left arm while the distal end of the right arm has been swung laterally apart from the left after the trigger has been actuated to the down position;
Figure 10 is an enlarged, partial view of the device depicted in Figure 9, but with the trigger and handle top removed for clarity;
Figures 11A, 11B, 11C, 11D, and 11E are distal end views of the present invention depicting the sequence shown in Figures 3, 5, 7, 9, and 3 again, respectively;
Figures 12A, 12B, 12C, and 12D are alternate views of Figures 2 and 4, 6, 8, and 10, respectively;
Figures 13A, 13B, 13C, and 13D are cut-away views of Figures 12A, 12B, 12C, and 12D, respectively;
Figures 14A, 14B, and 14C are isometric views of the barrel for the clockwise, center, and counterclockwise positions, respectively;
Figure 15 is a enlarged, isometric view of the barrel depicted in Figure 14B, but with the barrel cover removed for clarity;
Figure 16 is a isometric view of the barrel depicted in Figure 14B, but with the barrel cover and yoke removed for clarity;
Figures 17A, 17B, and 17C are alternate views of the barrel depicted in Figures 14A, 14B, and 14C, respectively, with the barrel cover and yoke removed for clarity, and looking from the proximal end;
Figure 18 is an isometric view of a curved, surgical needle in the distal end of the present invention as depicted in Figure 5;
Figure 19 is an isometric view of a curved, surgical needle in the distal end of the present invention in the configuration corresponding to Figure 20A;
Figures 20A and 20B are top, cross-sectional views of the barrel as depicted in Figures 14A and 14B;
Figure 21 is a side, cross-sectional view of the barrel together with the loading button in the up position as depicted in Figure 2 for the present invention;
Figure 22 is a side, cross-sectional view of the barrel together with the loading button in the down or actuated position and corresponds to the present invention as depicted in Figure 1;
Figure 23 is an exploded, isometric view of all the components of the present invention:
Figure 24 shows isometric views of the present invention as it may be held by a surgeon and depicting a method for its use;
Figure 25 is a isometric view of a curved, surgical needle with flat sides held in the distal end of an alternate embodiment of the present invention;
Figure 26 is a cutaway view of the distal end of one arm of the present invention holding a curved, surgical needle with flat sides; and
Figure 27 is a top sectional view of the distal end of one arm of the present invention holding a curved, surgical needle with flat sides.

The drawings are not necessarily to scale.

### DETAILED DESCRIPTION OF THE INVENTION

The suturing device of the present invention can be used to suture any type of anatomical tissue which may also be sutured using the traditional surgical methods described earlier. The device described herein is for use through any sufficiently-sized incision into the body, such as a mini-thoracotomy, but may also be used on external portions of the body such as for plastic surgery. It should also be clear to one of ordinary skill in the art that the present invention can be configured and provided with sealing means so as to make it possible to use through an endoscopic port. The device described herein is shown being used with a surgical needle having a particular radius of curvature. It may also be used with needles having other curvature radii or of many other shapes and sizes, including ski-shaped and straight needles, depending on the surgeon's preference for the surgical procedure being performed.

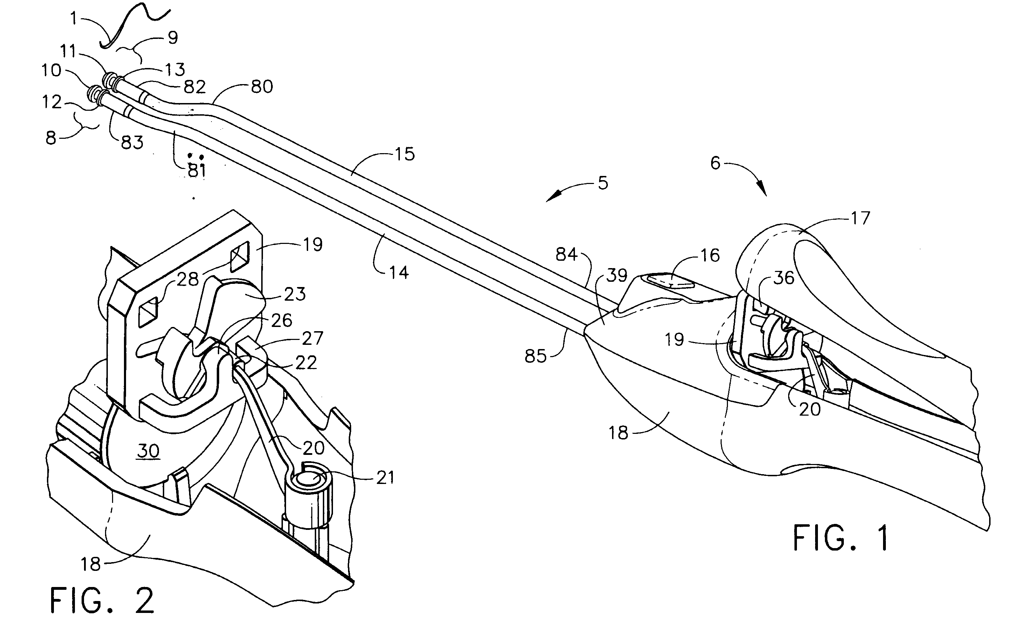

Referring now to the figures wherein like numerals indicate the same elements throughout the views there is shown in Figure 1 a suturing device 5 in accordance with the present invention. Device 5 includes a handle 6 at its proximal end. Handle 6 includes a button 16 and a trigger 17 whose functions will be explained in detail below. Handle 6 is essentially a cylindrical body which is shaped ergonomically so that a surgeon may comfortably grip it and actuate the button 16 and trigger 17 with the same hand. The handle top 39, the handle bottom 18, the trigger 17, and the button 16 can be made of any substantially rigid, medical grade material but is preferably formed of a plastic such as polycarbonate. As will become apparent, this one hand manipulation of the device is advantageous in that it frees the surgeon's other hand for holding the tissue or other instrumentation. The length of the trigger 17 and the general shape of the handle 6 may vary from what is depicted in Figure 1 in order to accommodate particular ergonomic requirements.

Extending distally from the handle 6 is a left tubular arm 14 and a right tubular arm 15. While the arms 14 and 15 are preferably round tubular, substantial longitudinal portions of them may have other cross-sectional shapes such as rectangular tubular or a C-shaped channel. The distal portions of arms 14 and 15 each have an offset bend section 80, 81 or dogleg so that the longitudinal axis of the distal arm ends 82, 83 are parallel to and offset from the longitudinal axis of the proximal arm ends 84, 85. The distal arm ends 82, 83 may differ from what is shown in that one or both of them may also be slightly angled with respect to the longitudinal axis of the device rather than be parallel to it. For example, both distal arm ends could be equally angled in towards each other. On the distal tip of left arm 14 is left gripper 8 comprising a left gripper head 10 and the left arm flange 12. Similarly, the distal tip of right arm 15 has a right gripper 9 comprising a right gripper head 11 and the right arm flange 13. As will be explained in greater detail below, grippers 8 and 9 are designed to alternately grip and release needle 1. as it is being passed from right arm 15 to left arm 14 and back again. The gripper heads 10, 11 and the arms 14, 15 may be made of a rigid, medical grade material including metal and plastic, but the preferred material is stainless steel.

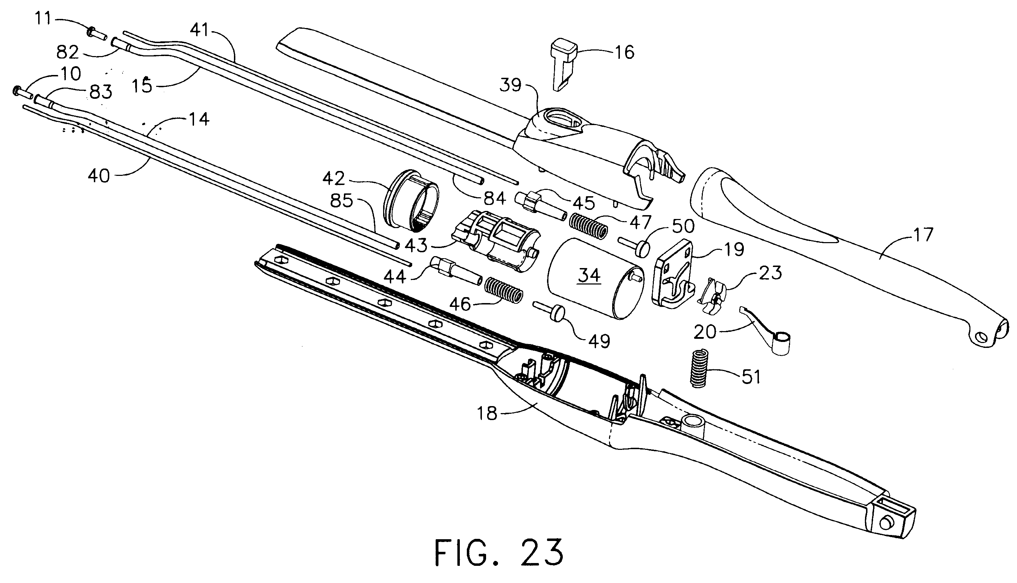

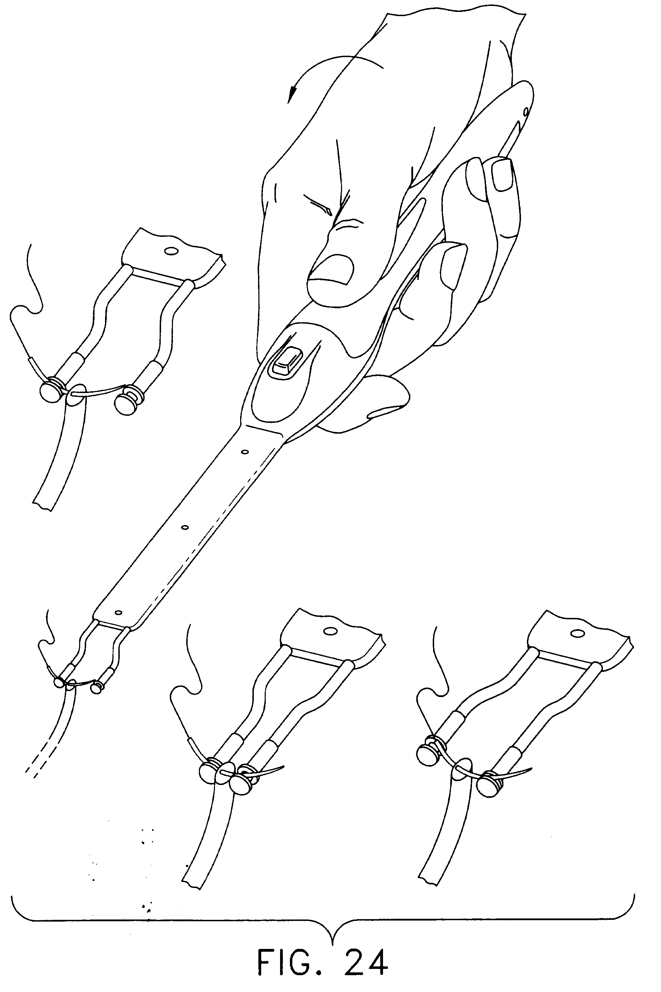

The proximal portion of the arms 14, 15 may be exposed as shown in Figures 1, 3 ,5 ,7 , and 9, or may also be covered partially by the handle top 39 and handle bottom 18 as depicted in Figures 23 and 24. The length of the arms 14, 15 may vary considerably without resulting in a change of the function or usage of the device. Both of these variations are limited mostly by the overall rigidity of the device and the ease of manipulating it.

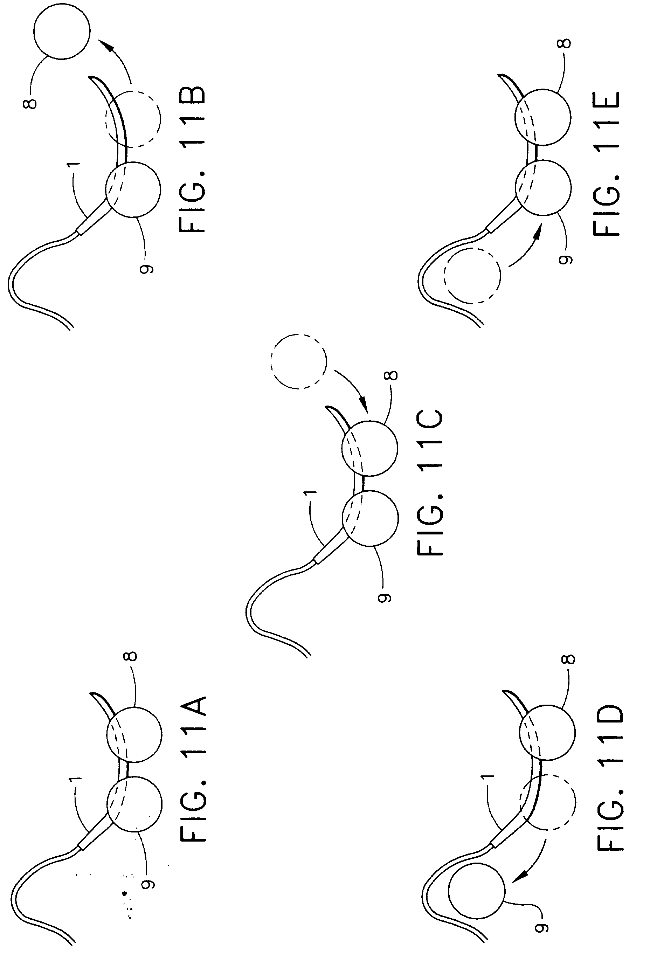

A brief and simplified description of how the device 5 works can best be accomplished by referring to Figures 11A-11E in conjunction with Figure 1. In Figure 11A the needle has just been loaded into the device so that both grippers 8 and 9 are holding the needle and the trigger 17 is in its up position (the trigger is explained in greater detail below). Thereafter, trigger 17 is pressed down and left gripper 8 releases the needle and swings away from the distal end of arm 15 as shown in Figure 11B. The arc length of this swing motion is the same for each full actuation. However, it is possible to vary the design of the mechanisms later described so that this arc length can be significantly more or less than what is shown in Figure 11B. The needle 1 can now be inserted into tissue and brought back out again by the surgeon's own action of twisting his wrists or the like. (This method of suturing using the present invention is depicted in Figure 24.) Now right gripper 9 is on the side of the needle insertion site and left gripper 8 is on the side of the needle exit site. Therefore, the trigger is now released to its up position again so that left gripper 8 can grip the needle again as shown in Figure 11C. As the trigger is pressed down this time, it is the right gripper 9 which releases the needle and is swung away from the left gripper 8 as shown in Figure 11D. The arc length of this motion may be designed also to vary significantly as described for the other gripper. In this position the surgeon can now move the needle away from the tissue, pulling the suture therethrough so as to complete a stitch. Once this is done the trigger is again released so that gripper 9 grips the needle again and the process can be repeated. As will become apparent, the proximal arm ends 84, 85 never translate laterally with respect to each other. The swinging back and forth action of the left and right grippers is accomplished by axially rotating the arms 14, 15, causing the offset bend sections 80, 81 to move with respect to the handle 6.

The detail of how the above sequence of operation is accomplished by device 5 can best be described by referring to Figures 1, 3, 5, 7, and 9. Each of these figures has a corresponding illustration, Figures 2,4,6,8, and 10 respectively, showing an enlarged portion of the mechanism in the handle 6 which controls the opening and closing of the grippers 8 , 9 and the rotation of the arms 14, 15. Hereinafter, the needle will be described as being passed from the right gripper 9 to the left gripper 8 and them back to the right gripper 9, but it is also possible for the needle point to be directed in the opposite lateral direction, in which case the needle would be passed from the left gripper 8 to the right gripper 9 and then back to the left gripper 8. It should be noted also that the traditional surgical technique for pitching and catching a needle has been maintained in the present invention, in that a twisting motion of the surgeon's hand to penetrate the tissue with the needle as well as to pull the needle and trailing suture out of the tissue is still required. This is advantageous in that the surgeon still controls the precise placement and manipulation of the needle, yet is able to do so with one device rather than with two as when using traditional suturing techniques.

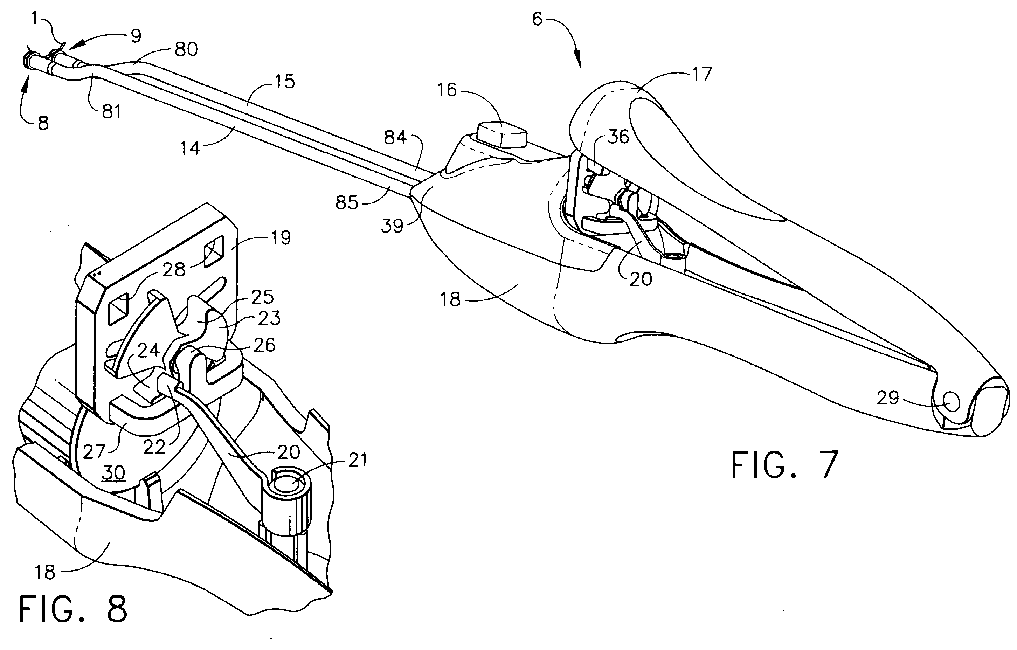

Referring to Figure 1, a surgical needle with trailing suture is shown in the position it would be held as it is lowered into the left and right grippers 8 and 9. During this step, the button 16 is depressed by the surgeon, causing both the left and right gripper heads 10, 11 to be displaced distally from the corresponding left and right arm flanges 12, 13, creating a sufficient gap to allow loading of the needle. The actual mechanism for moving the left and right gripper heads will be explained in detail below. As will become apparent to those of ordinary skill in art as they read this description, the button 16 can also be pushed in order to load the needle 1 when the trigger is in the position shown in Figure 7.

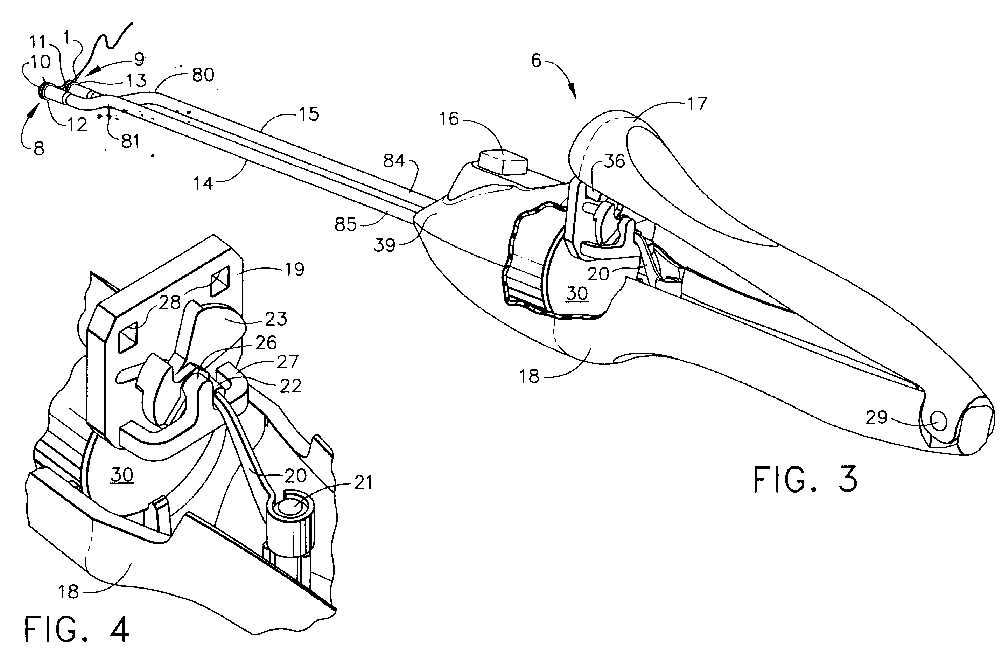

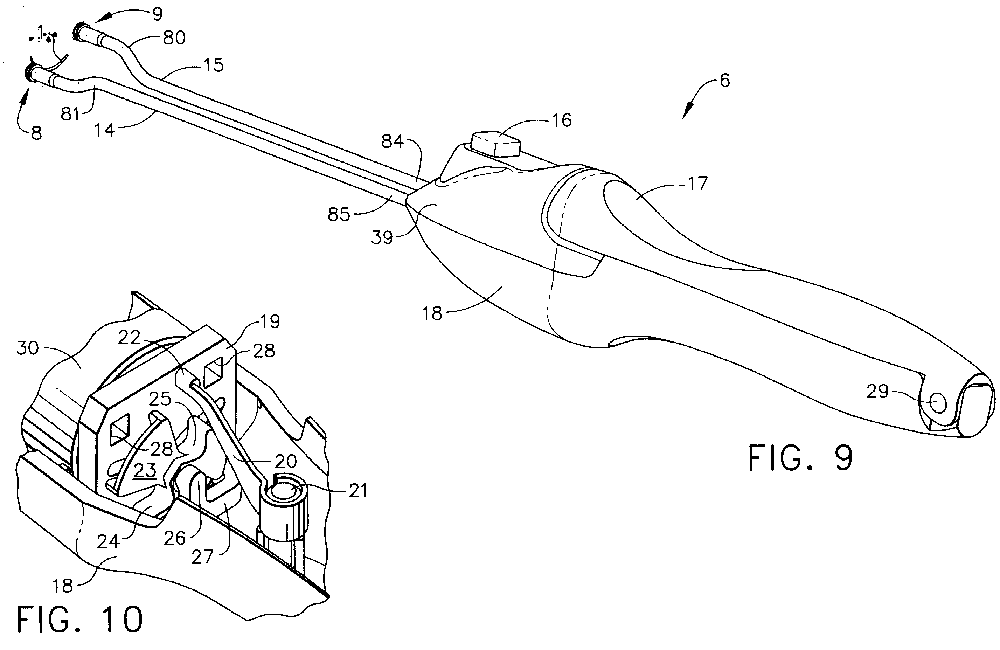

In Figures 1 and 2, the trigger 17 is not yet actuated and is in what is hereinafter referred to as its "up" position. Figure 2 shows an enlarged view of some of the components of the handle and trigger mechanisms, with trigger 17 and handle bottom 39 removed for clarity. Two trigger prongs 36 (partially visible in Figure 1, 3, and 7) project distally from the bottom side of trigger 17. These prongs fit slidably into toggle plate recesses 28 of toggle plate 19. When the trigger is actuated in the downward direction, pivoting about trigger pivot 29, the toggle plate and the trigger distal end move vertically together. The trigger is biased in the "up" position by trigger spring 51 (see Figure 23.) In Figures 1, 2, since the trigger is in the "up" position for this step, the toggle plate 19 is also in the "up" position with respect to handle 6. Handle 6 further includes a toggle 23 which is pivotably attached to the toggle plate 19 and partially resides within a toggle plate rail 27. Toggle 23 has a left toggle surface 24 and a right toggle surface 25 (see Figures 8, 10.) In Figure 4, toggle 23 is shown in its full, clockwise position.

Handle 6 further includes a toggle switch 20 mounted to handle bottom 18 by toggle switch mount 21. Toggle switch 20 has a tip 22, which is shown in Figure 2 as bearing against right toggle surface 24 and toggle plate rail 27. Toggle switch 20 is attached to switch mount 21, and flexes in the lateral direction. However, it does not move up or down with respect to the handle. It is toggle plate 19 that moves up and down with respect to the handle. In Figure 2, toggle switch 20 is flexed to its right-of-center position. Also partially visible in Figure 2 is cylindrical barrel 30 nested in the bottom handle 18. As will be explained later, it is the rotation of this barrel about its longitudinal axis which causes the movement of the grippers 8, 9, and the arms 14, 15.

The toggle plate 19 and toggle 23 are preferably made of a rigid, medical grade plastic such as polyetherimide. The toggle switch 20 functions as a leaf spring biased in the central plane position which intersects the pivot 26 of the toggle 23 and is made of a spring material such as a rigid plastic or preferably, stainless steel.

Referring now to Figure 3, the button 16 has been released so that the left and right gripper heads 10, 11 move proximally under spring force to hold the needle tightly against the left and right arm flanges 12, 13. The position of the left and right grippers as shown is hereinafter referred to as the "home" position. As can be seen. Figures 2 and 4 are identical because the trigger has not been actuated, only button 16 has been deactuated. Therefore, the toggle plate 19, toggle 23, and toggle switch 20 have not changed position.

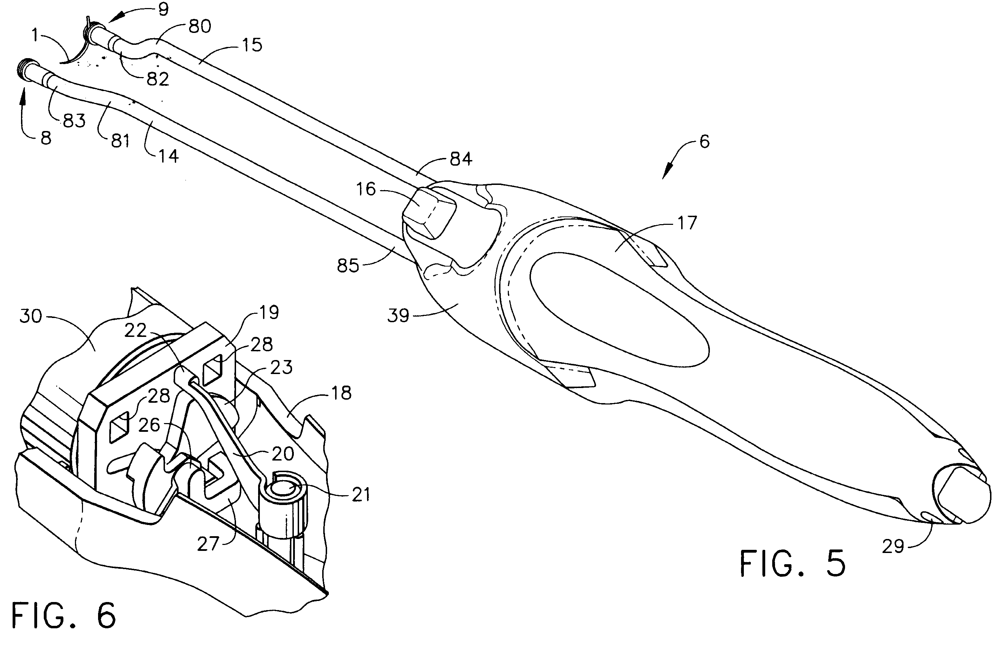

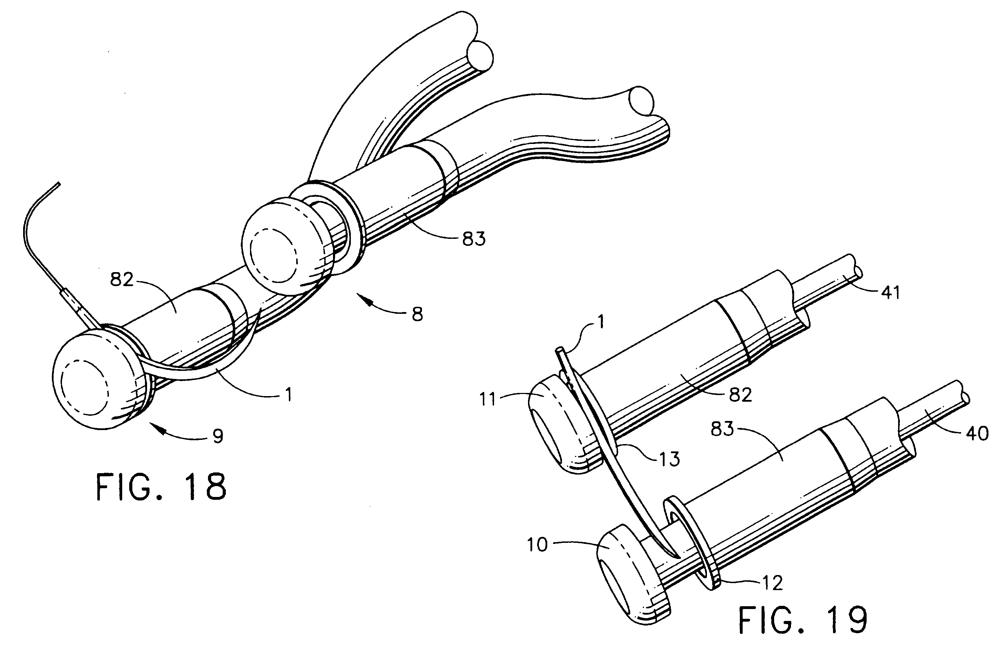

Now that the surgeon has loaded the needle into the grippers 8 and 9, device 5 can be further explained by referring to Figure 5. In this figure, the trigger has been pressed down by the surgeon, so that the trigger is now in what is hereinafter referred to as its "down" position. In this figure, the entire device has been rotated slightly about its longitudinal axis for clarity. By placing the trigger in its down position, the needle 1 has been released by left gripper 8 and arm 14 has rotated or spun about its longitudinal axis so that left arm distal end 83 has swung away from the right arm distal end 82. Figure 18 shows a clear, perspective view of the distal portion of the present invention shown in Figure 5. In this view it can be seen that the left gripper 8 has swung away from its home position and is open, thus allowing the surgeon to penetrate the tissue with the needle 1.

By referring to Figure 6, one can see the movement of the toggle plate 19 and toggle 23. When the trigger is pressed down by the surgeon, the toggle plate 19 moves down within handle bottom 18, carrying toggle 23 with it. As a result, toggle plate rail 27 has moved down and away from toggle switch tip 22, allowing toggle switch 20 to spring laterally to its biased center position.

At this point in the sequence of operation, the needle is ready to be placed into the tissue using the same surgical technique as when using a conventional needle driver. While the trigger 17 is still being held down, the needle tip is placed against the tissue and the handle 6 is twisted clockwise about its longitudinal axis so that the needle tip penetrates the tissue and exits on the opposite side. This step is depicted in Figure 24.

After the surgeon has penetrated the tissue he needs to pull the suture all the way through to make a stitch. This is done by returning the trigger to its up position and can best be described by referring to Figure 7. As seen from that figure, the trigger 17 has been released to its up position so that the left gripper 8 has returned to its home position to grasp the needle 1, while right gripper 9 has maintained its grasp of needle 1, much in the way that it did in Figure 3. However, by referring to Figure 8, one can see that the toggle 23 and toggle switch 20 are in different positions than that shown in Figure 4. As seen from this Figure, the toggle plate 19 has again moved upwardly with the trigger 17, carrying with it toggle 23. However, during this movement, toggle switch tip 22 now contacts left toggle surface 24 (see Figure 4), causing toggle 23 to rotate counter clockwise as the trigger 17 is released to the up position.

The next step of the operational sequence is the actuation of the trigger 17 to its down position again, as shown in Figure 9. This causes the right gripper 9 to release the needle and causes arm 15 to rotate so that its distal end swings away from the left gripper 8. In this configuration, it is now possible for the surgeon to use the device like a conventional needle holder and to pull the needle 1 and its trailing suture (see the third sequence of Figure 24) out of the tissue, thus completing the stitch through tissue. Corresponding Figure 10 shows the movement of the toggle plate 19 to its down position, allowing the toggle switch 20 to spring to its center biased position. The final step of the operational sequence is to release trigger 17 and is done after the distal end of the device is moved away from the tissue in order to allow the return of right gripper 9 to its home position as shown in Figure 3. The toggle plate 19, the toggle 23, and the toggle switch 20 return also to the configuration shown in Figures 3 and 4, and the sequence may be repeated for another stitch into tissue.

Again, Figures 11A - 11E show an end view of the left and right grippers 8, 9 and the needle 1 for each of the steps of the operational sequence shown in Figures 1, 5, 7, 9 and 3 again, respectively. For each step, the needle 1 is stationary with respect to the device handle 6; it is always one of the grippers 8, 9 which is moving to and from the needle 1.

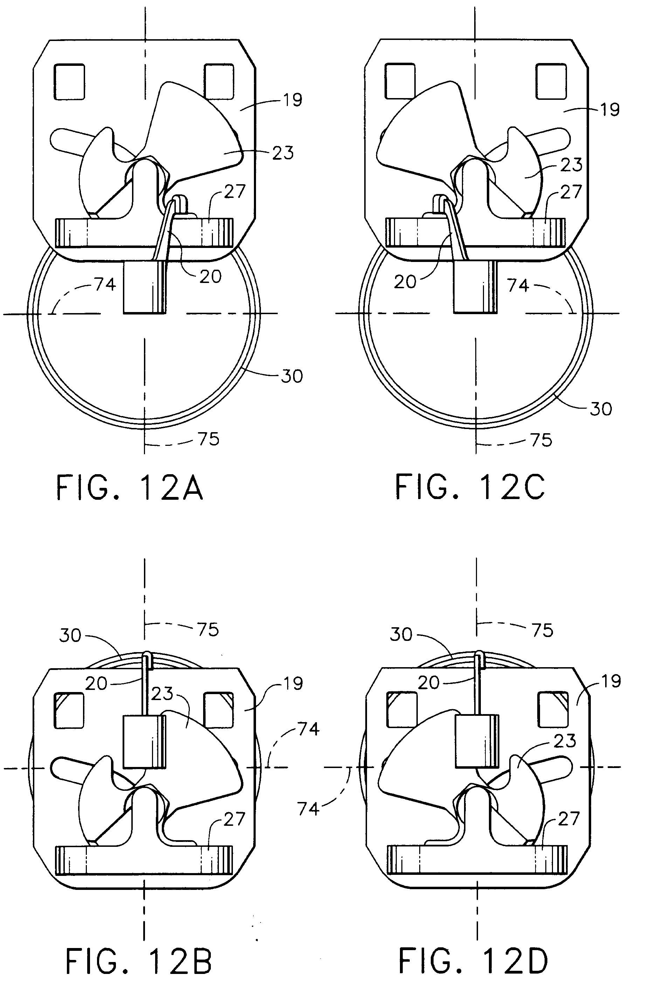

Figures 12A, 12B, 12C, and 12D are alternate views of Figures 2, 6, 8, 10, respectively. The toggle plate 19, toggle 23, switch 20, and barrel 30 are shown from the proximal end, and again depict the operational sequence. Note that for each step, barrel 30 remains centered on the X, Y reference frame axes 74, 75. It is the toggle plate 19 and toggle 23 which move vertically, causing toggle switch 20 to deflect laterally to the left or right of its centered position. Barrel 30 rotates either clockwise or counterclockwise due to its interaction with the up and down movement of the toggle plate 19. As will be explained below, rotation of the barrel causes the rotation of the arms 14 and 15 and the simultaneous actuation of grippers 8 and 9. The barrel as shown rotates upon the inside of the handle bottom 18. It should be appreciated that there are various other features that may be designed into the handle and/or onto the barrel in order to provide rotational support for the barrel, and that the embodiment shown is not intended to be limiting.

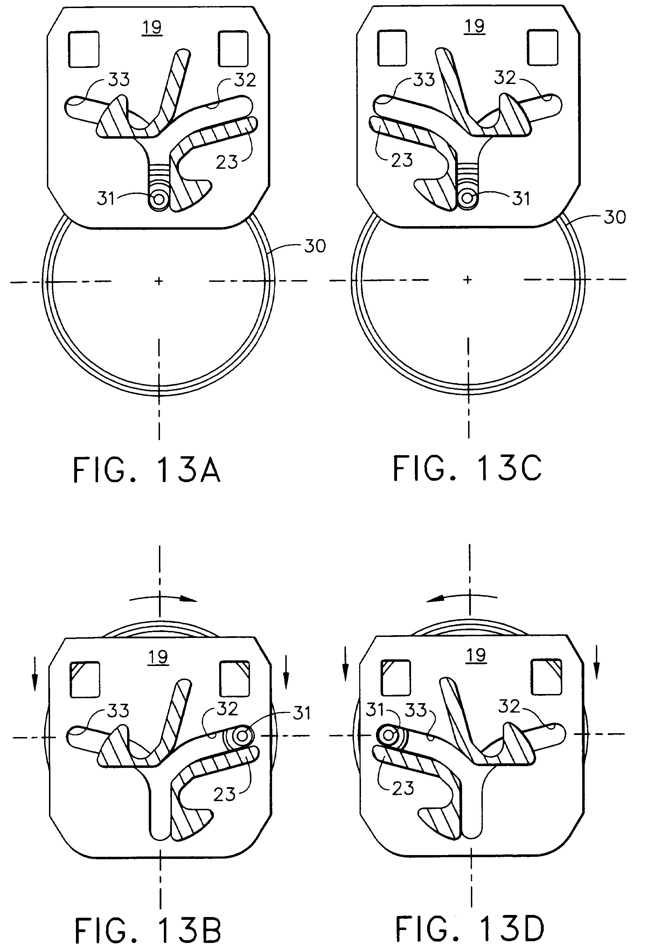

As seen from Figures 13A - 13D, when the trigger is first actuated into the down position the barrel rotates clockwise about 90 degrees. When the trigger is released back into the up position, the barrel rotates counter clockwise about 90 degrees back to its original position. Thereafter, when the trigger is again fired the barrel then rotates counter clockwise about 90 degrees and when released it rotates clockwise back to its original position. Figures 13A, 13B, 13C, and 13D correspond to Figures 12A, 12B, 12C, and 12D, respectively. The rotation of the barrel is accomplished by the fact that the barrel is connected to toggle plate by projection 31 and left and right toggle plate tracks 33, 32. When trigger 17 is actuated, the full clockwise orientation of the toggle 23 allows the barrel projection 31 to traverse the right toggle track 32 (Figure 13B) while the full counterclockwise orientation of the toggle allows the barrel projection 31 to traverse the left toggle track 33 (Figure 13D).

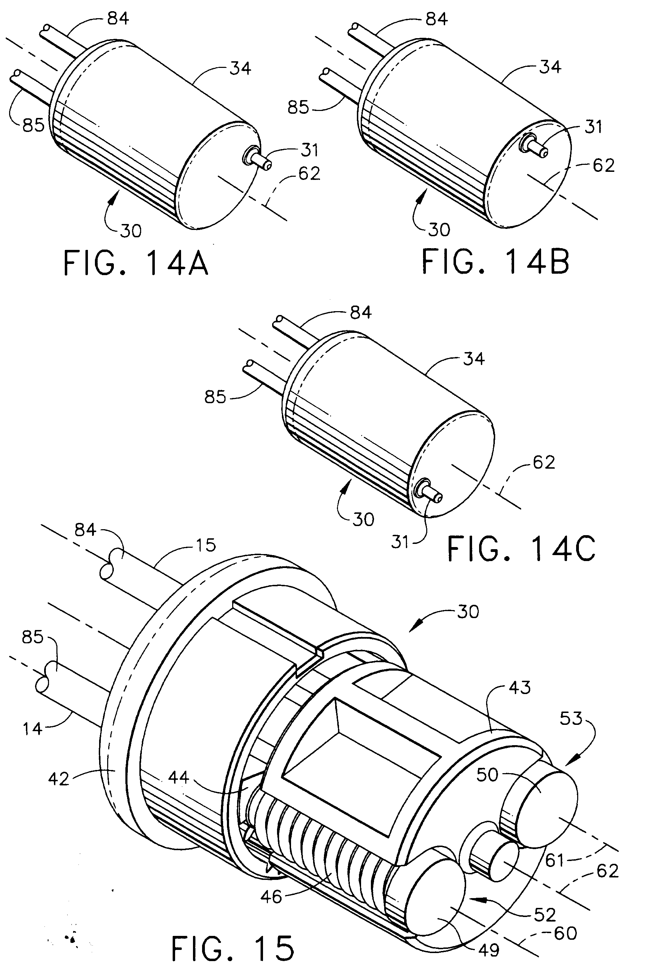

Figures 14A, 14B, and 14C show the three possible positions of the barrel projection 31 due to the interaction with the toggle plate 19 and the toggle 23 for when the trigger 17 is in the fully upward or fully downward position. Figure 14A shows the full clockwise rotation about the longitudinal axis 62; Figure 14B shows the center or "home" position of the barrel; Figure 14C shows the full counterclockwise position. Partial views of the left arm proximal end 85 and the right arm proximal end 84 entering into the distal end of the barrel 30 are shown. As seen from these figures, the left and right arm proximal ends remain in a constant position with respect to each other. As mentioned before, movement of distal arm ends is caused by the rotation of the arms acting upon the offset bend sections 80, 81.

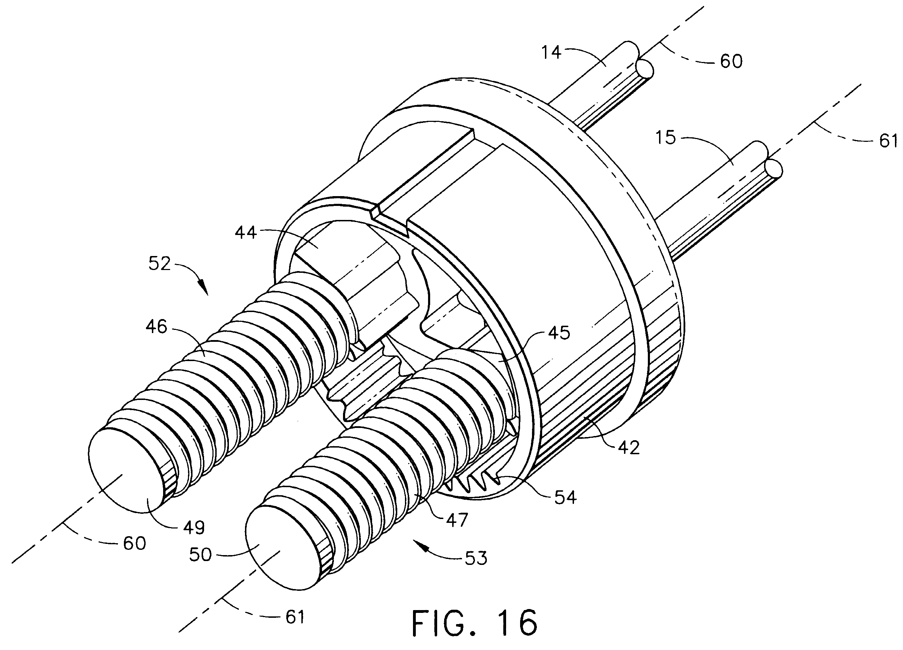

Figure 15 corresponds with Figure 14B and is a perspective view of the assembled components inside the barrel 30. Barrel cover 34 has been removed for clarity. Barrel 30 includes a yoke 43, preferably made from a rigid plastic. Cam lid 42 is made from a rigid plastic and fits tightly together with the barrel cover 34 to encapsulate the assembly. Yoke 43 houses a left pinion assembly 52 and a right pinion assembly 53. The following components of the left pinion assembly 52 are individually depicted in the exploded view of Figure 23: a left pinion 44 which is attached to left arm proximal end 85; a left cam head 49 which may be metal or plastic and fits slideably into left pinion 44 and captures left pinion spring 46. Likewise, the right pinion assembly 53 includes a right cam head 50, right pinion spring 47 (not shown in Figure 15), a right pinion 45 (also not shown in Figure 15) and right arm proximal end 84. Figure 16, wherein the yoke has been removed, has been presented to show the left and right pinion assemblies 52, 53 respectively as they interact with the inside of cam lid 42. Cam heads 49, 50 are attached to cables 40, 41, respectively, which are assembled slideably through longitudinal holes in pinions 44, 45.

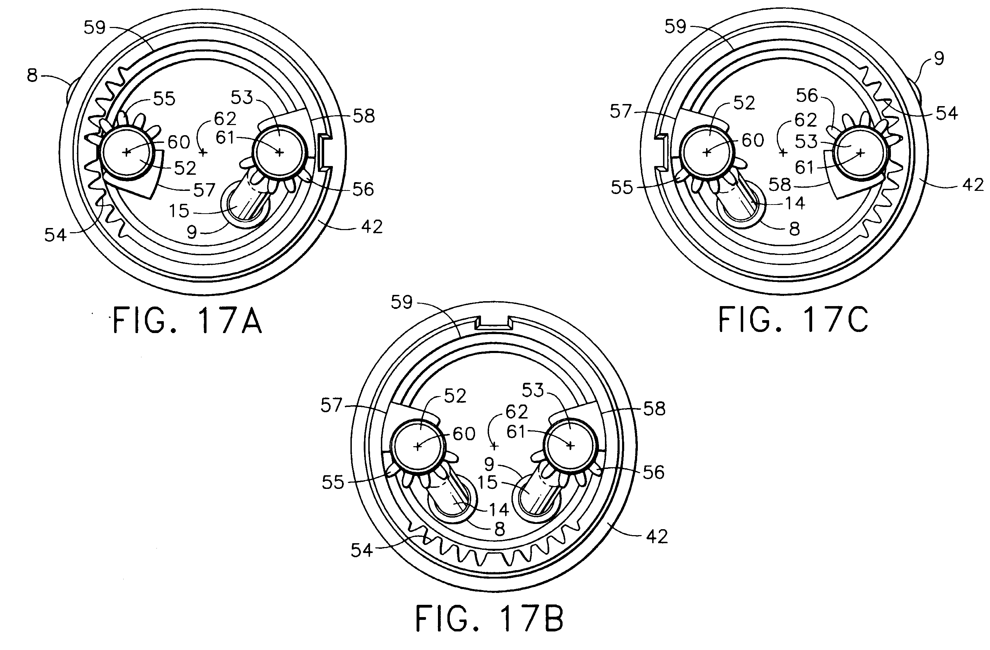

Figures 17A, 17B, 17C correspond with Figures 14A, 14B, and 14C, respectively, and show the proximal end of the components shown in Figure 16. These figures are intended to show how the rotation of the barrel 30, and hence cam lid 42 due to their attachment to one another, causes the rotation of the left and right pinion assemblies 52, 53, which in turn causes the arms 14, 15 to rotate in order to swing grippers 8, 9 between their two end positions. In Figure 17A, the cam lid 42 has been rotated to the full clockwise position due to the trigger actuation and the steps described in the preceding paragraphs. This cam lid position corresponds to Figures 5, 6, 12B, and 13B.

The cam lid gear teeth 54 mesh with the left pinion gear teeth 55 to cause the rotation of the pinion assembly 52 about its longitudinal axis 60 as the trigger is preseed into its down position. The rotation of pinion assembly 52 about its longitudinal axis 60 causes arm 14 to rotate which in turn causes left gripper 8 to swing to the position shown in Figures 5 and 11B. Simultaneously, right pinion sliding surface 58 of the right pinion assembly 53 slides along cam lid sliding surface 59, the right pinion assembly 53 therefore does not rotate about axis 61, and the right gripper 9 stays in its home position. The opposite interaction takes place when the cam lid 42 is caused to rotate counterclockwise as shown in Figure 17C, causing right gripper 9 to swing away from its home position. Figure 17B depicts the home position for both grippers 8, 9 and occurs when the cam lid gear teeth 54 are located in the central position and neither the left pinion gear teeth 55 or the right pinion gear teeth 56 are engaged.

Before the rotation of the left arm 14 takes place, causing the left gripper 8 to swing away from its home position, it is necessary for the left gripper 8 to release its hold of the needle 1, while the right gripper 9 maintains its grip. (See Figure 19.) This is also true for the when the right arm 15 swings away from its home position, that is, the right gripper 9 must first release the needle 1. The timing for the actuation of grippers 8, 9 is designed accordingly, and is controlled by the cam mechanism within the barrel 30.

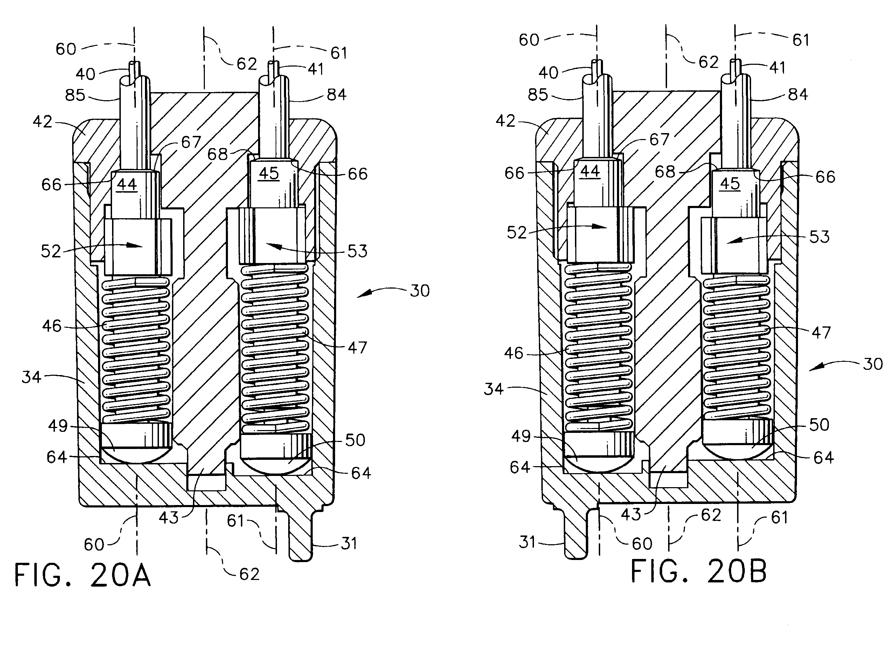

Figures 20A and 20B are longitudinal cross-sectional views of the barrel 30, cam lid 42, and yoke 43, containing the left and right pinion assemblies 52, 53, as configured in Figures 14A and 14C respectively, where the section for each was taken through a plane intersecting main axis 62 and barrel projection 31. Referring first to Figures 20A and 19, left arm proximal end 85 is attached to left pinion assembly 52 and right arm proximal end 84 is attached to right pinion assembly 53. Left and right pinion assemblies 52, 53 are shown in their relative positions within the barrel and are comprised of left and right cam heads 49, 50, and left and right pinion springs 46, 47, respectively. The left cam head 49 is attached to a left cable 40, which runs through the entire length of arm 14 where it is attached at its distal end to left gripper head 10, whereby all three components move together. Likewise, the right cam head 50 is attached to a right cable 41 which is attached to right gripper head 11 so that these three components move together. Compression springs 46 and 47 exert a longitudinal force such that cables 40 and 41 are usually, unless as otherwise noted below, under tension.

Cables 40, 41 are flexible in order to move easily in the longitudinal direction through the offset of the distal portion of the left and right arms 14, 15 respectively, and may be made of a semi-rigid plastic or of metal wire strands. The cables are constrained in the channels within the arms so that they also can transmit a compressive force sufficient for moving the gripper heads distally.

In Figures 20A , 20B it can be seen that the barrel 30 has a peripheral cam surface 64 at its proximal end. Cam surface 64 is always in contact with the left and right cam heads 49 and 50, and the right and left pinion cam surfaces 67, 68 are always in contact with the cam lid peripheral cam surface 66, due to the longitudinally outward force of the left and right pinion springs 46 and 47.

In Figure 20A the barrel is rotated in the full clockwise position, as also shown in Figures 6, 12B, 13B, 14A, and 17A. The right pinion spring 47 is assembled into the barrel partially compressed and is shown in Figure 20A for when it has its maximum allowable length. The longitudinal force of the spring has caused the right cam head 50 to move proximally, thereby moving cable 41 and right gripper head 9 proximally. Simultaneously, right pinion 53 has moved distally, causing right arm 15 and right arm flange 13 to move distally. These two, opposing movements provide a bi-directional grip on the needle 1. Again in Figure 20A, the left pinion spring 46 is at its minimum allowable length because the left arm 14 was moved in the proximal direction and the left cam head 49 was moved in the distal direction. This results in the bi-directional separation of the left gripper head 10 and the left arm flange 12 as can be seen in Figure 19. This separation feature is advantageous because the needle is centered on the gap between the gripper head 10 and arm flange 12, and therefore allowable variation of needle alignment with the gap is greater than if only the gripper head 10 or the arm flange 12 were to move alone. This feature facilitates "catching" of the needle. Figure 20B shows the opposite configuration to that of Figure 20A and corresponds to Figure 14C for when the barrel 30 is rotated to the full counterclockwise position. The needle is gripped in the left gripper 8 and released by right gripper 9, just prior to right arm 15 swinging away from the home position.

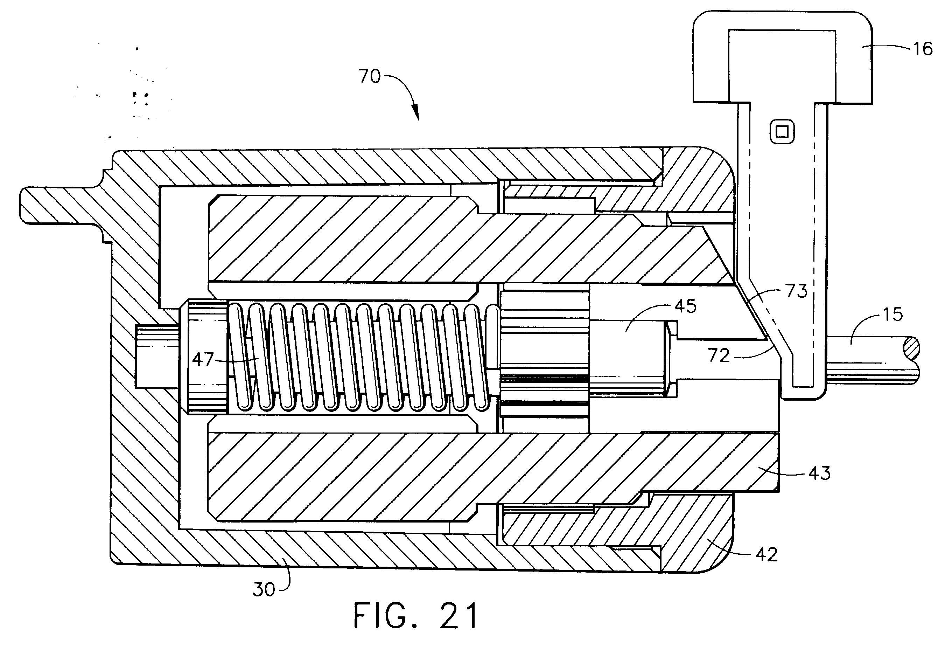

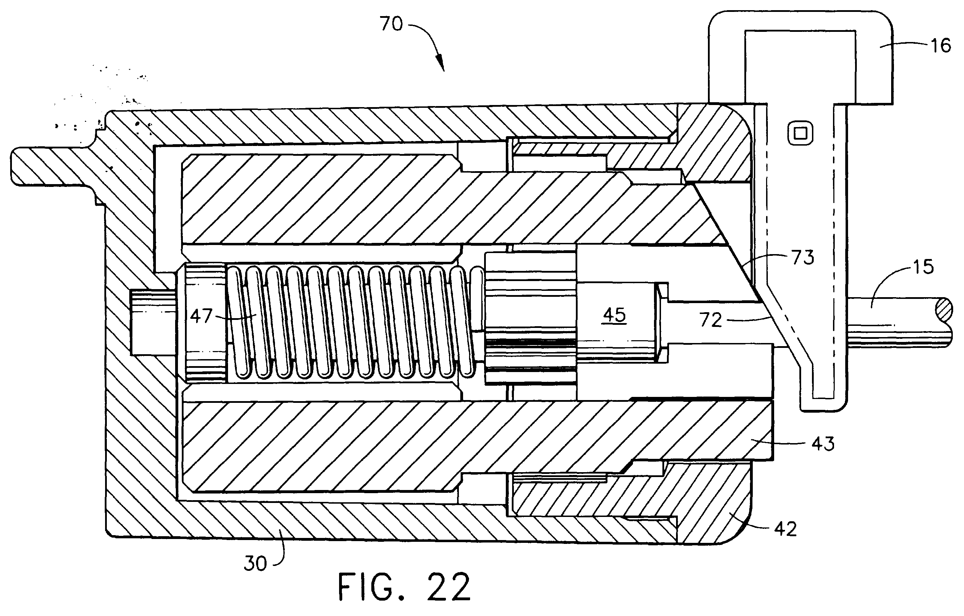

Now turning to Figures 21 and 22, the operation of the button 16 for the loading and release of needle 1 from the distal end of the present invention is next described. In Figure 21, which corresponds to Figure 3, the needle 1 is gripped by both the left gripper 8 and the right gripper 9, both in the home position, the button 16 is in the full upward or non-actuated position. It is held there by the spring forces exerted by the left and right pinion springs 46, 47 pushing simultaneously against left and right cam heads 49, 50, and against the left and right pinions 44, 45, which in turn impart a longitudinally directed force on the yoke 43 in the distal direction. A yoke ramp surface 73 transmits this force to the button ramp surface 72, forcing the button 16 vertically upwards. In Figure 22 which corresponds to Figure 1 when the needle 1 may be loaded into or released from the grippers 8, 9, the button 16 is pushed downwards by the surgeon, imparting a longitudinal force in the proximal direction onto the yoke 43, which in turn bears against left and right pinions 44, 45, causing them to move in the proximal direction against the spring forces exerted by left and right pinion springs 44, 45, and finally resulting in the movement in the proximal direction of left and right arms 14, 15 which are attached to left and right pinions 44, 45. Thus, the respective gaps between the gripper heads 10, 11 and the arm flanges 12, 13 increase equally to a width greater than the diameter of the needle being used. The interfacing button ramp surface 72 and yoke ramp surface 73 also serve to key the yoke 43 so that it does not rotate when the trigger 17 is actuated and the barrel 30 and cam lid 42 rotate within the stationary handle bottom 18 and handle top 39.

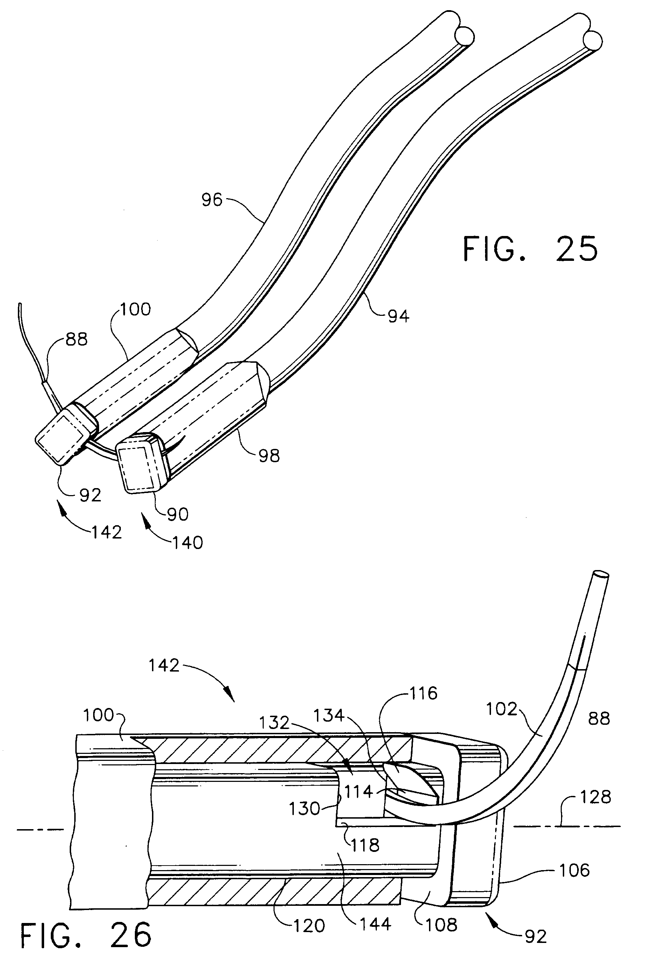

An alternate embodiment of the distal portion of the present invention is depicted in Figure 25. The remainder of the surgical device is the same as the previous embodiment. A curved, surgical needle 88 is shown being held by both a left gripper 140 and a right gripper 142. The right gripper 142 includes a right plunger 92 (which may also be referred to as a second opposing member) and a right opposing member 100 (which may also be referred to as a first opposing member). Right opposing member 100 is the distal portion of a tubular, right arm 96 and is formed into a rectangular cylinder. Right plunger 92 fits slidably into an aperture 144 of right opposing member 100, and aperture 144 is surrounded by a perimeter wall 134 (see Figure 26). The left gripper 90 mirrors the right gripper 92. In this embodiment, the curved needle 88 is shown with flattened sides to facilitate clamping and alignment in the grippers, 90 and 92. This alternate embodiment of the device may also be used with non-flattened needles, but with less effectiveness.

Turning next to Figure 26, the curved needle 88 is shown being held by the right gripper 142 which has been partially cut away for clarity. The plunger 92 includes a shank 120 extending slidably into right opposing member 100 and a hooking recess 132 defined by a shank wall 130, a shelf 118, a clamping member 114, and an inclined surface 116. A flange 106 is on the distal end of shank 120 and has an alignment surface 108. Clamping member 114 is slightly proximal and approximately parallel to alignment surface 108. The curved needle is held between clamping member 114 and a perimeter wall 134 of right opposing member 100. As can be seen in this figure, the curved needle 88 is trapped within the hooking recess 132 and is held upright; that is, the longitudinal axis 128 of opposing member 100 is approximately normal to the plane defined by the curved needle 88. The curved needle may be placed into or out of the hooking recess 132 when the gripper flange 106 is separated sufficiently from perimeter wall 134, during the sequence of operation of the device. The curved needle 88 is held in the proper orientation for suturing through tissue by the steps already described. The reason the curved needle self-orients into this configuration and why this alternate embodiment also allows for a more secure grip on the needle 88 than in the previous embodiment is described next.

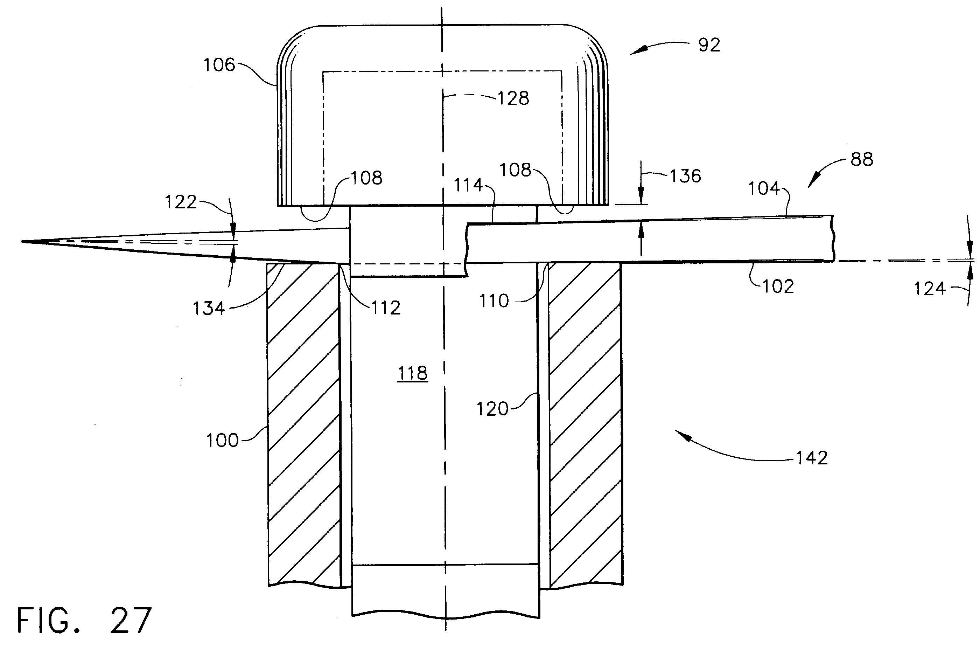

Referring now to Figure 27, a top view of a longitudinal cross section of the right opposing member 100 and the right plunger 92 is shown with the tapered portion of the curved surgical needle 88 clamped in place. (The features described for the right gripper 142 are identical to those of the left gripper 140.) The curved needle 88 includes a first and second flattened surface, 102 and 104, respectively. First flattened surface 102 is held against perimeter wall 134 at first and second locations, 110 and 112, respectively, which are spaced laterally apart and approximately equidistant from longitudinal axis 128. Clamping member 114 of plunger 92 clamps against second flattened surface 104 of curved needle 88, and on a portion of the curved needle laterally between the first and second locations, 110 and 112. This clamping method represents the needle 88 as a simply loaded beam, causing minute angular deflections 122 and 124. The magnitude of these deflections depends on the force applied by the plunger 92, but is easily maintained in the elastic region of the needle deformation so that release of the needle by the plunger 92 allows the needle to immediately restraighten. By clamping onto the needle 88 in this manner, the resistance of the needle to lateral slip during suturing into tissue is significantly increased as compared to the previous embodiment.

Still referring to Figure 27, an alignment gap 136 is shown between flange proximal surface 108 and the second flattened surface 104 of curved needle 88. This gap is provided so that clamping member 114 can bear against the curved needle 88, but is sized minimally (about .002 inches) so that the curved needle 88 is forced to be oriented uprightly as already described. This gap 136 is approximately equal to the height of the clamping member 114 over the flange proximal surface 108. If the gap 136 is too large, the curved needle 88 would tend to rotate about its axis approximately 90 degrees to what is shown in Figure 26, with the needle ends pointing distally, as the plunger 92 is closed against the needle. This is not the desired orientation for suturing with the device and is prevented by minimizing the size of the gap 136. It is desired that the plane of the needle 88 stay within an angular range of 0-10 degrees from the plane to which the longitudinal axis 128 of the gripper 92 is normal. Other predetermined angles of needle orientation could be established by the proper selection of the size of the gap 136. In general, the larger the gap, the further distal the ends of the needle would point.

From the foregoing discussion, it is clear to those skilled in the art that the clamping action of other embodiments of the present invention can be reversed. That is, the plunger 92 could be made to be stationary and the opposing member 100 could be made to move distally, thus gripping the needle therebetween.

Although particular embodiments of the present invention have been shown and described, modification may be made to the device without departing from the spirit and scope of the present invention. The terms used in describing the invention are used in their descriptive sense and not as terms of limitations.

## Claims

1. A device for assisting a physician in suture procedures using a needle and a suture attached thereto, said device comprising:
a) a handle for holding said device, said handle having distal and proximal ends;
b) right and left arms extending distally from said handle, said arms having proximal ends attached to said handle and distal ends having grippers attached thereto for gripping and releasing a needle, each of said grippers comprising first and second opposing members which move closely adjacent to one another to grip said needle, said first opposing member comprising an aperture at least partially surrounded by a perimeter wall, said second opposing member comprising a clamping member, extending therefrom, opposing said aperture, said clamping member is sized such that it can be at least partially inserted within said aperture, whereby when said gripper is activated, said perimeter wall presses against a first side of said needle at two spaced apart locations, and said clamping member presses against a second opposing side of said needle, between said two spaced apart locations; and
c) at least one means for moving said distal ends of said arms closely adjacent to one another and for passing said needle from one gripper to the other and thereafter for moving said distal ends of said arms further apart from one another.

2. The device according to claim 1, further including a needle gripped by at least one of said grippers, wherein said first and second sides of said needle are substantially flat.

3. The device according to claim 1 wherein said needle has distal and proximal ends and is curved therebetween.

4. The device according to claim 3, wherein when one of said grippers grips said needle, said distal and proximal ends of said needle rotate between zero to ten degrees towards said distal end of said arm.

5. The device according to claim 3 wherein when one of said grippers grips said needle, said distal and proximal ends of said needle rotate between zero to forty five degrees towards said distal end of said arm.

6. The device according to claim 1 wherein said clamping member extends from said second opposing member a distance no greater than 0.01 inch.
